# EUROPÄISCHE PATENTSCHRIFT

(11) **EP 0 727 196 B1**
(45) Veröffentlichungstag und Bekanntmachung des Hinweises auf die Patenterteilung: **07.10.1998**
(21) Anmeldenummer: 95120302.5
(22) Anmeldetag: 21.12.1995
(51) Int. Cl.: A61F 2/44

(54) **Platzhalter, insbesondere für einen Wirbel bzw. eine Bandscheibe**
Device for reserving space, especially for a vertebra or for a spinal disc
Dispositif pour garder un espace, en particulier pour un vertébré ou pour un disque intervertébré

(30) Priorität: 14.02.1995 DE 19504867
(43) Veröffentlichungstag der Anmeldung: 21.08.1996
(73) Patentinhaber: Harms, Jürgen, Prof. Dr., 76337 Waldbronn (DE); Biedermann, Lutz, 78048 Villingen-Schwenningen (DE)
(72) Erfinder: Harms, Jürgen, Prof. Dr., 76337 Waldbronn (DE); Biedermann, Lutz, 78048 Villingen-Schwenningen (DE)
(74) Vertreter: Prüfer, Lutz H., Dipl.-Phys.

(56) Entgegenhaltungen:
- EP-A- 0 369 603
- EP-A- 0 517 030
- EP-A- 0 529 275
- EP-A- 0 566 810
- US-A- 5 192 327

## Beschreibung

Die Erfindung betrifft einen Platzhalter nach dem Oberbegriff des Patentanspruches 1.

Ein solcher Platzhalter ist beispielsweise aus der EP-A-0 268 115 bekannt. Dieser weist auf seiner Innenseite in einem Abstand von dem jeweiligen freien Ende des Mantels einen durch einen Ring gebildeten Anschlag auf. Der Ring ist mittels Schrauben mit dem Mantel verbunden. In einer besonderen Ausführungsform ist auf dem Ring eine Durchbrechungen aufweisende Bodenplatte aufgebracht.

Aufgabe der Erfindung ist es, den Platzhalter einfacher und gleichzeitig universeller auszugestalten.

Diese Aufgabe wird durch den in Patentanspruch 1 gekennzeichneten Platzhalter gelöst.

Ausführungsformen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren. Von den Figuren zeigen:
- Fig. 1: eine Seitenansicht des Mantels des Platzhalters;
- Fig. 2: eine Draufsicht auf eine erste Ausführungsform des mit dem Mantel zu verbindenden Elementes;
- Fig. 3: eine Draufsicht auf eine zweite Ausführungsform des Elementes;
- Fig. 3a: einen Schnitt entlang der Linie IIIA-IIIA in Fig. 3;
- Fig. 4: eine Draufsicht auf eine dritte Ausführungsform des Elementes;
- Fig. 5: eine Schnittdarstellung einer Seitenansicht entlang der Linie V-V in Fig. 4;
- Fig. 6: eine Schnittdarstellung entlang der Linie VI-VI in Fig. 7 durch eine weitere abgewandelte Ausführungsform;
- Fig. 7: eine Draufsicht auf diese Ausführungsform,
- Fig. 8: eine Schnittdarstellung entlang der Linie VIII-VIII in Fig. 9;
- Fig. 9: eine Draufsicht auf diese weitere Ausbildungsform;
- Fig. 10: eine Schnittdarstellung entlang der Linie X-X in Fig. 11; und
- Fig. 11: eine Draufsicht auf diese weitere Ausbildungsform.

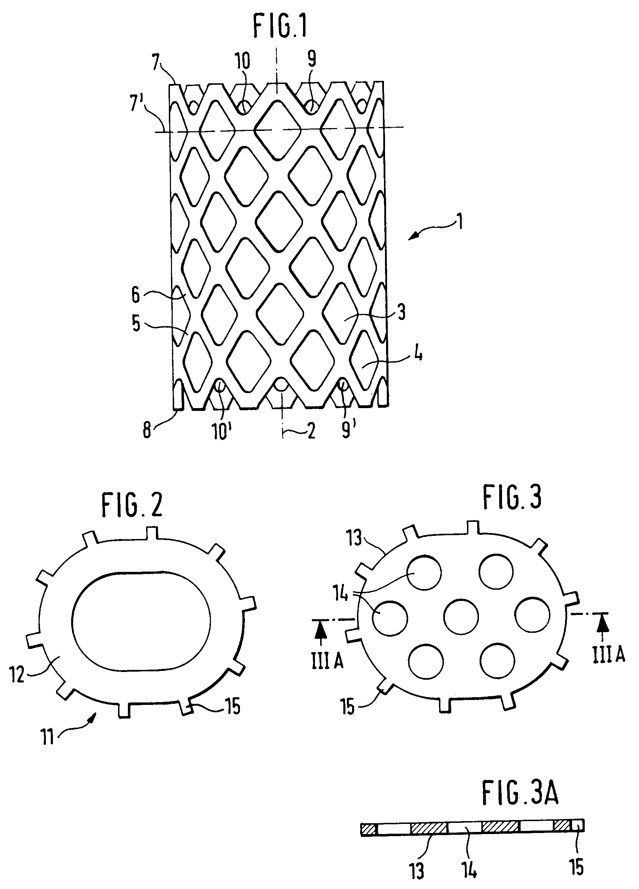

Wie insbesondere aus Fig. 1 ersichtlich ist, weist der Platzhalter einen geschlossenen Mantel 1 auf. Der sich senkrecht zur Längsachse 2 des Mantels 1 erstreckende Querschnitt ist in bekannter Weise insbesondere zylindrisch oder oval oder nierenförmig ausgebildet. Der Mantel 1 weist in der aus Fig. 1 ersichtlichen Weise sich mit ihrer Längsdiagonale parallel zur Mantelachse 2 erstreckende rautenförmige Ausnehmungen 3, 4 auf. Jeweils benachbarte Reihen solcher Rauten 3, 4 sind gegeneinander um eine halbe Rautenhöhe versetzt. Dadurch wird ein Netz von sich unter einem spitzen Winkel schneidenden Bandstreifen 5, 6 gebildet, die unter jeweils gleich großen Winkeln gegen die Längsdiagonale der Rauten 3,4 geneigt sind. Der obere Rand 7 und der untere Rand 8 erstrecken sich jeweils in einer Ebene senkrecht zu der Längsachse 2. Die Größe der Rauten 3, 4 und der diese begrenzende Bandstreifen 5, 6 ist so gewählt, daß die Anzahl von Rauten in Umfangsrichtung stets ganzahlig ist. Durch das Bilden der Ränder 7, 8 ergibt sich in Umfangsrichtung stets eine gerade Anzahl von durch den jeweiligen Rautengrund gebildeten V-förmigen Vertiefungen 9, 10 bzw. 9', 10'. Aufgrund der wie oben beschrieben gewählten Geometrie weist der jeweilige Rand quasi eine zu einem Punkt auf der Längsachse 2, der in der Ebene des Randes liegt, bestehende Punktsymmetrie auf.

Das in Fig. 2 gezeigte erste Ausführungsbeispiel eines einen Anschlag bildenden Elementes 11 ist als ein plattenförmiger Ring ausgebildet. Der Ring 12 entspricht in seiner Außenkontur der Innenkontur des Mantels 1. Seine Abmessungen sind so gewählt, daß er sich in das Innere des Mantels hineindrücken, gewünschtenfalls aber auch wieder herausdrücken läßt, also durch Reibsitz mit dem Mantel 1 verbunden ist. An dem Außenrand des Ringes 12 sind in Umfangsrichtung äquidistant verteilt hervorspringende Stege 15 vorgesehen. Der Abstand zweier Stege in Umfangsrichtung ist gleich dem Abstand zweier in Umfangsrichtung aufeinander folgender V-förmiger Vertiefungen 9, 10. Die Querabmessungen der Stege 15 in der Plattenebene sind so bemessen, daß die Stege gut in den Grund der V-förmigen Ausnehmungen 9, 10 hineinpassen. Die Länge der hervorstehenden Stege entspricht in etwa der Wanddicke des zugehörigen Mantels.

Im Fall der Anwendung wird der Mantel 1 durch Abtrennen des oberen Randes 7 und des unteren Randes 8 auf die gewünschte Länge gebracht. Dann wird ein Ring 12 auf der Oberseite und ein zweiter Ring auf der unteren Seite so in das Mantelinnere gedrückt, daß der jeweilige Ring mit seinen Stegen 15 in den jeweiligen Grund der zugehörigen V-förmigen Vertiefung 9, 10 bzw. 9', 10' liegt.

Aufgrund der Tatsache der ganzzahligen Anzahl der V-förmigen Vertiefungen und der sich daraus ergebenden Punktsymmetrie kann ein und dasselbe Element 11 verwendet werden, unabhängig davon, ob die Abtrennung an dem Rand 7 oder an dem gestrichelt angedeuteten darunterliegenden Rand 7' erfolgt. Wird der Rand nicht an der Stelle 7 sondern an der Stelle 7' gebildet, wird der Ring 12 einfach um seine Längsachse gedreht eingesetzt, so daß die Lagerhaltung vereinfacht wird und andererseits auch die Handhabung sehr einfach ist, weil nur eine Art von Ringen zum Einsatz kommt.

Fig. 3 zeigt ein Element 13 einer abgewandelten Ausführungsform. Auch hier handelt es sich um eine Platte, bei der die Ausnehmungen durch in der Platte verteilte lochförmige Ausnehmungen 14 gebildet sind. In allen übrigen Merkmalen stimmt das Element mit dem Element 11 überein.

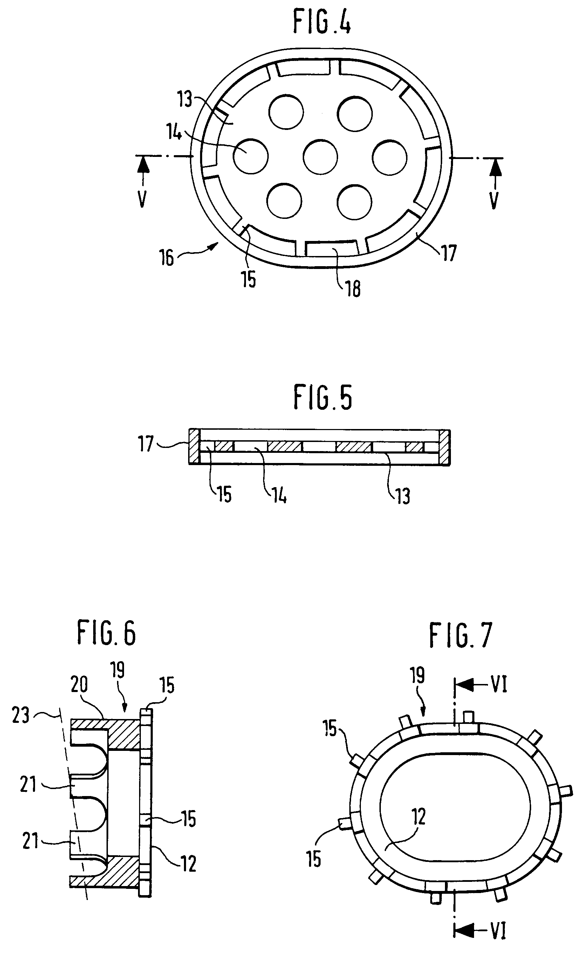

Die Figuren 4 und 5 zeigen ein Element 16 einer dritten-Ausführungsform. Dieses Element weist zunächst wiederum eine Platte auf, die mit der in Fig. 3 gezeigten Ausführungsform bezüglich ihrer Ausnehmungen 14 und ihrer Stege 15 identisch ist. Um die Platte herum ist ein Außenring 17 angeordnet. Dieser erstreckt sich in der am besten aus Fig. 5 ersichtlichen Weise mit seiner Ringwandung senkrecht zur Plattenebene und damit parallel zur äußeren Oberfläche des Mantels 1. Die Lange der Stege 15 ist so gewählt, daß diese gerade um so viel länger als die Dicke des Mantels 1 ist, daß der dadurch zwischen Platte, Stegen und Ring gebildete Zwischenraum 18 ein Aufschieben des Elementes auf das jeweilige freie Ende 7, 8 des Mantels 1 dahingehend, daß die Stege 15 in dem Grund der jeweiligen zugehörigen V-förmigen Ausnehmungen 9, 10, 9', 10' liegen, ermöglicht. Der Ring 17 liegt dann mit seiner inneren Oberfläche an der Außenwand des Mantels 1 an.

In den Figuren 6 und 7 ist ein Element 19 einer weiteren Ausführungsform beschrieben. Diese weist einen plattenförmigen Ring 12, der mit dem in Fig. 2 gezeigten Ring identisch ist. Auf einer Oberfläche dieses Ringes ist, wie am besten aus Fig. 6 ersichtlich ist, ein in seiner Außenkontur der Innenkontur des Mantels 1 entsprechender Randabschnitt 20 vorgesehen, dessen der Platte 12 abgewandter freier Rand in Umfangsrichtung äquidistant angeordnete Zacken 21 aufweist. Die Höhe der Zacken 21 über der Platte 12 ist so bemessen, daß die Zacken im eingesetzten Zustand nahezu bis zu ihrem Grund über den Rand 7 bzw. 8 des Mantels 1 hervorstehen.

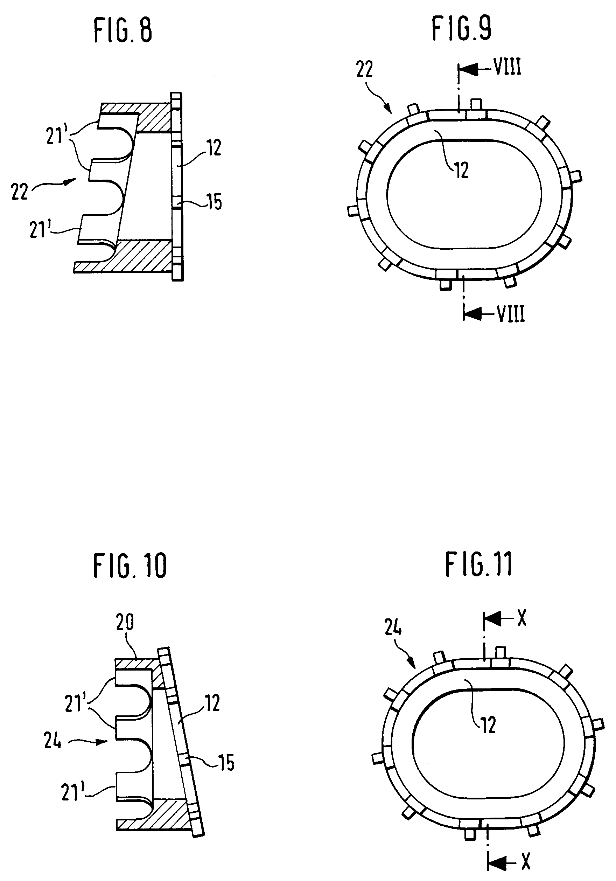

Die in den Figuren 8 und 9 gezeigte Ausführungsform eines Elementes 22 unterscheidet sich von der vorher beschriebenen Ausführungsform nur dadurch, daß der Zackengrund nicht auf einer Ebene parallel zur Platte 12 sondern in einer Ebene, die geneigt zur Platte 12 ist, liegt. Der von den Zacken gebildete Rand liegt ebenfalls in einer Ebene, die zu der Plattenebene des Ringes 12 geneigt ist. Die Neigung beträgt vorzugsweise 8 bis 10°.

Die in den Figuren 10 und 11 gezeigte Ausführungsform des Elementes 24 unterscheidet sich von der in den Figuren 8 und 9 gezeigten Ausführungsform dadurch, daß der Randabschnitt 20 schräg zu der Plattenebene, d.h. in einem von 90° verschiedenen Winkel zur Plattenebene des Ringes 12, ausgebildet ist.

Im Betrieb werden beide Ausführungsformen, also die Elemente 19 bzw. 22 wie bei den vorher beschriebenen Ausführungsformen in den Mantel eingesetzt, so daß der Ring 12 mit seinen hervorstehenden Stegen 15 in den tiefsten Ausnehmungen der V-förmigen Vertiefungen 9, 10 ruht und die Zacken über den Mantelrand nach außen hervorstehen. Die Zacken 21 bzw. 21' können bei der in Fig. 6 und 7 gezeigten Ausführungsform unterschiedlich lang abgeschnitten werden, beispielsweise entlang der gestrichelten Linie 23, so daß ein keilförmiger Einsatz entsteht. In gleicher Weise kann auch der Rand des Elementes 22 so beschnitten werden, daß der vorgegebene Winkel zwischen dem äußeren Rand und dem plattenförmigen Ring 12 verändert wird. Auf diese Weise ist es möglich, mit wenigen Grundelementen Platzhalter mit verschiedenen Keilwinkeln zu bilden.

Die Außenkontur der jeweiligen Ringe 12 bzw. 13 wird natürlich in Abhängigkeit von der jeweiligen Innenkontur des zugehörigen Mantels bestimmt.

In Abweichung von den oben beschriebenen besonders bevorzugten Ausführungsbeispielen können anstelle der V-Form die Vertiefungen des Randes auch andere Formen haben, etwa U-förmige oder schlitzförmige Vertiefungen.

## Patentansprüche

1. Platzhalter, insbesondere für einen Wirbel bzw. eine Bandscheibe, mit einem Ausnehmungen aufweisenden Mantel (1) mit einem ersten und zweiten Rand (7, 8) und in Umfangsrichtung angeordneten und zueinander benachbarten sich jeweils in Richtung des anderen Randes erstreckenden Vertiefungen (9, 10; 9', 10') und einem an wenigstens einem der Enden in einem Abstand von dem äußeren Rand vorgesehenen Anschlag, wobei der Anschlag durch ein Element (11, 13, 16, 19, 22) gebildet ist, dessen äußere Kontur der inneren Kontur des Mantels (1) entspricht,
dadurch gekennzeichnet, daß
am Umfang des Elementes (11, 13, 16, 19, 22) an den den Vertiefungen (9, 10; 9', 10') im Mantel (1) entsprechenden Stellen stegartige Vorsprünge (15) zum Eingreifen in die Vertiefungen vorgesehen sind.

2. Platzhalter nach Anspruch 1, dadurch gekennzeichnet, daß
die Vertiefungen als zueinander jeweils einen gleichen Abstand aufweisende V-förmige Ausnehmungen (9, 10; 9', 10') ausgebildet sind.

3. Platzhalter nach Anspruch 1 oder 2, dadurch gekennzeichnet, daß
die Abmessung der äußeren Kontur so bemessen ist, daß das Element (11, 13, 16, 19, 22) mit Reibsitz von dem Mantel (1) gehalten ist.

4. Platzhalter nach einem der Ansprüche 1 bis 3, dadurch gekennzeichnet, daß
der Mantel (1) aus einem Gitter mit rautenförmigen Ausnehmungen (3, 4) gebildet ist.

5. Platzhalter nach Anspruch 4, dadurch gekennzeichnet, daß
eine Diagonale der Rauten (3, 4) sich jeweils parallel zur Längsachse (2) des Platzhalters erstreckt.

6. Platzhalter nach einem der Ansprüche 1 bis 5, dadurch gekennzeichnet, daß
das Element (11, 13, 16) als Platte ausgebildet ist.

7. Platzhalter nach Anspruch 6, dadurch gekennzeichnet, daß
die Platte (12) ringförmig ausgebildet ist.

8. Platzhalter nach Anspruch 6, dadurch gekennzeichnet, daß
die Platte (13) mehrere einander benachbarte Löcher (14) aufweist.

9. Platzhalter nach einem der Ansprüche 6 bis 8, dadurch gekennzeichnet, daß
die Länge der Stege (15) wenigstens gleich der Wandstärke des Mantels (1) ist.

10. Platzhalter nach Anspruch 9, dadurch gekennzeichnet, daß
an den äußeren Enden der Stege (15) ein sich um das Element (13) herum erstreckender Außenring (17) vorgesehen ist, dessen Kontur der Außenkontur des Mantels (1) entspricht und dessen Ringwandung sich senkrecht zur Ebene die Platte (13) bzw. parallel zur äußeren Oberfläche des Mantels (1) erstreckt.

11. Platzhalter nach Anspruch 10, dadurch gekennzeichnet, daß
der Außenring (17) mit den Stegen (15) entlang seiner Mittellinie verbunden ist.

12. Platzhalter nach einem der Ansprüche 1 bis 11, dadurch gekennzeichnet, daß
auf der dem angrenzenden Ende zugewandten Seite des Elementes (19, 22) ein über den freien Rand des Mantels (1) hervorstehender zackenförmiger Rand (21, 21') vorgesehen ist.

13. Platzhalter nach Anspruch 12, dadurch gekennzeichnet, daß
der zackenförmige Rand sich parallel zu der Platte (12) erstreckt.

14. Platzhalter nach Anspruch 12, dadurch gekennzeichnet, daß
der zackenförmige Rand sich schräg zu der Platte (12) erstreckt.

15. Platzhalter nach einem der Ansprüche 1 bis 14, dadurch gekennzeichnet, daß
der Rand eine gerade Anzahl äquidistanter Vertiefungen aufweist.

## Claims

1. A spacer device, in particular for a vertebra or a vertebral disc, having a sheathing (1) which has recesses, a first and second edge (7, 8) and mutually adjacent depressions (9, 10; 9', 10') arranged in the peripheral direction and extending in each case in the direction of the other edge, and a stop provided at at least one of the ends at a spacing from the outer edge, the stop being formed by an element (11, 13, 16, 19, 22) whereof the outer contour corresponds to the inner contour of the sheathing (1), characterized in that, at the periphery of the element (11, 13, 16, 19, 22), web-type projections (15) for engaging in the depressions are provided at the points corresponding to the depressions (9, 10; 9', 10') in the sheathing (1).

2. A spacer device according to Claim 1, characterized in that the depressions are constructed as V-shaped recesses (9, 10; 9', 10') in each case having an equal spacing from one another.

3. A spacer device according to Claim 1 or 2, characterized in that the outer contour is dimensioned such that the element (11, 13, 16, 19, 22) is held with frictional fit by the sheathing (1).

4. A spacer device according to one of Claims 1 to 3, characterized in that the sheathing (1) is formed from a lattice having rhomboidal recesses (3, 4).

5. A spacer device according to Claim 4, characterized in that a diagonal of the rhombi (3, 4) extends in each case parallel to the longitudinal axis (2) of the spacer device.

6. A spacer device according to one of Claims 1 to 5, characterized in that the element (11, 13, 16) is constructed as a plate.

7. A spacer device according to Claim 6, characterized in that the plate (12) is of an annular construction.

8. A spacer device according to Claim 6, characterized in that the plate (13) has a plurality of mutually adjacent holes (14).

9. A spacer device according to one of Claims 6 to 8, characterized in that the length of the webs (15) is at least equal to the wall thickness of the sheathing (1).

10. A spacer device according to Claim 9, characterized in that an outer ring (17) extending about the element (13) is provided at the outer ends of the webs (15), the contour of which outer ring corresponds to the outer contour of the sheathing (1) and the annular wall of which outer ring extends perpendicularly to the plane of the plate (13) and parallel to the outer surface of the sheathing (1).

11. A spacer device according to Claim 10, characterized in that the outer ring (17) is connected along its centre line to the webs (15).

12. A spacer device according to Claims 1 to 11, characterized in that an indented edge (21, 21') projecting beyond the free edge of the sheathing (1) is provided on the side of the element (19, 22) which faces the adjoining end.

13. A spacer device according to Claim 12, characterized in that the indented edge extends parallel to the plate (12).

14. A spacer device according to Claim 12, characterized in that the indented edge extends obliquely with respect to the plate (12).

15. A spacer device according to one of Claims 1 to 14, characterized in that the edge has an even number of equidistant depressions.

## Revendications

1. Dispositif d'espacement, en particulier pour une vertèbre ou un disque intervertébral, comprenant une gaine (1) munie d'évidements, comportant un premier et un deuxième bord (7, 8) et des cavités (9, 10 ; 9', 10') disposées dans le sens périphérique et contiguës les unes aux autres, s'étendant respectivement en direction de l'autre bord, et une butée prévue au moins sur l'une des extrémités à une distance donnée du bord extérieur, la butée étant formée par un élément (11, 13, 16, 19, 22) dont le contour extérieur coïncide avec le contour intérieur de la gaine (1), caractérisé en ce qu'il est prévu de réaliser dans la gaine (1) sur le pourtour de l'élément (11, 13, 16, 19, 22) des saillies (15) en forme de nervures, qui correspondent aux cavités (9, 10 ; 9', 10') pour pouvoir s'engager dans lesdites cavités.

2. Dispositif d'espacement selon la revendication 1, caractérisé en ce que les cavités sont conçues sous forme de cavités en V (9, 10 ; 9', 10') disposées à égale distance les unes des autres.

3. Dispositif d'espacement selon la revendication 1 ou 2, caractérisé en ce que la dimension du contour extérieur est définie de telle sorte que l'élément (11, 13, 16, 19, 22) est maintenu par friction dans la gaine (1).

4. Dispositif d'espacement selon l'une quelconque des revendications 1 à 3, caractérisé en ce que la gaine (1) est formée par un réseau d'évidements (3, 4) en forme de losanges.

5. Dispositif d'espacement selon la revendication 4, caractérisé en ce qu'une diagonale des losanges (3, 4) s'étend toujours parallèlement à l'axe longitudinal (2) du dispositif d'espacement.

6. Dispositif d'espacement selon l'une quelconque des revendications 1 à 5, caractérisé en ce que l'élément (11, 13, 16) est conçu en forme de plaquette.

7. Dispositif d'espacement selon la revendication 6, caractérisé en ce que la plaquette (12) forme une bague.

8. Dispositif d'espacement selon la revendication 6, caractérisé en ce que la plaquette (13) est munie de plusieurs trous (14), contigus les uns aux autres.

9. Dispositif d'espacement selon l'une quelconque des revendications 6 à 8, caractérisé en ce que la longueur des nervures (15) est au moins égale à l'épaisseur de la paroi de la gaine (1).

10. Dispositif d'espacement selon, la revendication 9, caractérisé en ce qu'il est prévu de poser, autour de l'élément (13) sur les extrémités extérieures des nervures (15), une bague extérieure (17) dont le contour coïncide avec le contour extérieur de la gaine (1) et dont la paroi est perpendiculaire au plan de la plaquette (13) et parallèle à la surface extérieure de la gaine (1).

11. Dispositif d'espacement selon la revendication 10, caractérisé en ce que la bague extérieure (17) est assemblée aux nervures (15) le long de sa ligne médiane.

12. Dispositif d'espacement selon l'une quelconque des revendications 1 à 11, caractérisé en ce qu'il est prévu de réaliser, sur le côté de l'élément (19, 22), orienté vers l'extrémité voisine, un bord denté (21, 21') s'avançant en saillie au-dessus du bord libre de la gaine (1).

13. Dispositif d'espacement selon la revendication 12, caractérisé en ce que le bord denté est parallèle à la plaquette (12).

14. Dispositif d'espacement selon la revendication 12, caractérisé en ce que le bord denté est incliné par rapport à la plaquette (12).

15. Dispositif d'espacement selon l'une quelconque des revendications 1 à 14, caractérisé en ce que le bord comporte un nombre pair de cavités équidistantes.
